# EUROPEAN PATENT APPLICATION

(11) **EP 4 840 418 A1**
(43) Date of publication of application: **18.11.2026**
(21) Application number: 26178821.0
(22) Date of filing: 14.05.2026
(51) Int. Cl.: A61B 34/10

(54) **SYSTEM FOR SPINO-PELVIC PLANNING FROM 2D IMAGES**

(30) Priority: 16.05.2025 US 202519210568
(71) Applicant: Globus Medical, Inc., Audubon, PA 19403 (US)
(72) Inventor: CAMERON, Hayden, Philadelphia (PA), 19129 (US); CHAPPUIS, Olivier, 1095 Lutry (VD) (CH); DUCH, Loris, 1053 Bretigny-sur-Morrens (VD) (CH)
(74) Representative: Morabito, Sara

(57) **Abstract**

Disclosed are a method and system for planning a total hip arthroplasty (THA) procedure on a patient based on spino-pelvic alignment and mobility parameters. In some cases, a method includes: obtaining a pre-operative 2D image of the patient in each of a standing position and a seated position; evaluating a change in sacral slope (SS) in the 2D images from the standing position to the seated position, to assign a spinal mobility classification of: normal spine mobility (A), or stiff spine mobility (B); evaluating a Pelvic Incidence-Lumbar Lordosis (PI-LL) angle mismatch on the 2D image of the standing position, to assign a spine alignment classification of: normal spine alignment (1), or flat back deformity alignment (2); and planning placement of an acetabular shell prosthesis based on the spinal mobility classification and the spine alignment classification.

## Description

### CROSS-REFERENCE TO RELATED APPLICATIONS

The present application is a continuation of U.S. Patent Application No. 19/210,528, filed on May 16, 2025, which is incorporated herein in its entirety.

### BACKGROUND

The disclosure relates generally to computer-assisted devices, systems, and methods for use in computer-assisted, navigated surgical procedures including total hip arthroplasty (THA) procedures, and more particularly, to such devices, systems, and methods for planning and performing a THA procedure considering spino-pelvic alignment.

Hip arthroplasty, or hip replacement, is a surgical procedure used to resurface and reconstruct a hip joint that has been damaged by disease or injury, e.g., by arthritis or a fracture. THA devices replace both the acetabulum and the femoral head that collectively comprise the hip joint. An acetabular implant is secured to the acetabulum, forming a replacement articulating surface which interfaces with the femoral implant secured to the end of the femur. The femoral implant is pivotably coupled to the acetabular implant, thereby reconstructing the hip joint. Exemplary acetabular implants are disclosed in, e.g., US Patent Application No. 17/024,876, filed September 18, 2020 (published as US 2022/0087823 A1), which is incorporated by reference as though fully set forth herein.

Robotic surgery systems including computer-assisted navigation have become a well-established technique in operating rooms, including their use in arthroplasty procedures. Computer-assisted navigation systems provide surgeons with computerized visualization of how a surgical instrument or other device that is posed relative to a patient correlates to a pose relative to medical images of the patient's anatomy, and how those poses correlate to a pre-operative surgical plan. The surgical robot has a serial arm on which an end effector is mounted. The surgeon (or "user") may hold the end effector or any instruments coupled thereto, to perform surgical operations while watching in real time on a navigation system (e.g., on stand-alone display(s) or an Augmented Reality (AR) headset), and to receive various types of relevant feedback and information associated with a defined plan for and/or progress of the surgical procedure.

Various workflows are available for use with the system, which may incorporate, e.g., pre-operative scans or images of the patient (e.g., x-ray or Computerized Tomography (CT)). These workflows may be used to plan the implant position and orientation with respect to patient anatomy, and to navigate the robot and surgical instruments during the surgical procedure.

Although current surgical approaches offer sophisticated frameworks for planning implant placement such as, e.g., acetabular shell prosthesis placement, improved frameworks are needed to mitigate the risk of post-operative hip instability, dislocation, and revision, particularly in patients with lumbar fusion, spinal deformity, or abnormal spino-pelvic mobility.

### BRIEF DESCRIPTION OF THE INVENTION

A first aspect of the disclosure provides a method for planning a total hip arthroplasty (THA) procedure on a patient, comprising obtaining a preoperative 2D image of the patient in each of a standing position and a seated position; evaluating a change in sacral slope (SS) in the 2D images from the standing position to the seated position, to assign a spinal mobility classification of: normal spine mobility (A), or stiff spine mobility (B); evaluating a Pelvic Incidence-Lumbar Lordosis (PI-LL) angle mismatch on the 2D image of the standing position, to assign a spine alignment classification of: normal spine alignment (1), or flat back deformity alignment (2); and planning placement of an acetabular shell prosthesis based on the spinal mobility classification and the spine alignment classification.

In certain embodiments, the pre-operative 2D images are lateral views.

In certain embodiments, the spinal mobility classification of normal spine mobility (A) reflects a change in SS of at least 10° from the standing position to the seated position; and the spinal mobility classification of stiff spine mobility (B) reflects a change in SS of less than 10° from the standing position to the seated position.

In certain embodiments, the spinal alignment classification of normal spine alignment (1) reflects a PI-LL ± 10°, and the spinal alignment classification of flat back deformity alignment (2) reflects a PI-LL greater than (>) 10°.

Certain embodiments further comprise assigning to the patient a combined classification based on the spinal mobility classification and the spine alignment classification, wherein the combined classification is selected from: 1A, 1B, 2A, and 2B.

Certain embodiments further comprise, where the combined classification is 1A or 2A, planning the placement by defining a default orientation of the acetabular shell prosthesis by an inclination angle of 40° and an anteversion angle of 20-25°, based on the patient's combined classification.

Certain embodiments further comprise, where the combined classification is 1B, planning the placement by defining a default orientation of the acetabular shell prosthesis by an inclination angle of 45° and an anteversion angle of 25-30°, based on the patient's combined classification.

Certain embodiments further comprise, where the combined classification is 2B, evaluating a Posterior Pelvic Tilt on the 2D image of the standing position, to assign a posterior pelvic tilt classification of less than or equal to (≤) 13°, or greater than (>) 13°.

Certain embodiments further comprise planning the placement by defining a default orientation of the acetabular shell prosthesis by an inclination angle of 40° and an anteversion angle of 20-25°, based on the patient having the combined classification of 2B and the posterior pelvic tilt of less than or equal to (≤) 13°, or defining a default orientation of the acetabular shell prosthesis by an inclination angle of 45° and an anteversion angle of 25-30°, based on the patient having the combined classification of 2B and the posterior pelvic tilt of greater than (>) 13°.

Certain embodiments further comprise processing the pre-operative 2D images of the patient on a computer platform by identifying bone landmarks on the pre-operative 2D images; analyzing the pre-operative 2D images; and displaying results of the analyzing on a display in communication with the computer platform.

Certain embodiments further comprise identifying the bone landmarks automatically using a trained neural network, or manually identifying the landmarks on the pre-operative 2D images.

Certain embodiments further comprise identifying the bone landmarks by manually confirming or correcting a location of each of the bone landmarks.

Certain embodiments further comprise analyzing the pre-operative 2D images by drawing two or more anatomical reference lines based on the bone landmarks, and measuring angular measurements between the reference lines.

Certain embodiments further comprise analyzing the pre-operative 2D images by determining a spino-pelvic alignment parameter from the preoperative 2D images.

In certain embodiments, the spino-pelvic parameter comprises a spino-pelvic tilt (SPT) angle, and the 2D images are lateral views of the patient in the standing position and the seated position.

In certain embodiments, the spino-pelvic parameter comprises a pelvic incidence (PI) angle, and the 2D images are lateral views of the patient in the standing position and the seated position.

In certain embodiments, the spino-pelvic parameter comprises a sacral slope (SS) angle, and the 2D images are lateral views of the patient in the standing position and the seated position.

In certain embodiments, the spino-pelvic parameter comprises an anterior pelvic plane tilt (APPT) angle, and the 2D images are lateral views of the patient in the standing position and the seated position.

In certain embodiments, the spino-pelvic parameter comprises pelvic incidence-lumbar lordosis (PI-LL) angle mismatch, and the 2D images are lateral views of the patient in the standing position and the seated position.

Certain embodiments further comprise using the spino-pelvic alignment parameter as an input for planning the placement of the acetabular shell prosthesis.

A second aspect provides a system for planning a navigated total hip arthroplasty (THA) procedure on a patient, comprising a computer platform including a processor and a memory, operative to obtain a pre-operative 2D image of the patient in each of a standing position and a seated position; evaluate a change in sacral slope (SS) in the 2D images from the standing position to the seated position, to assign a spinal mobility classification of: normal spine mobility (A), or stiff spine mobility (B); evaluate a Pelvic Incidence-Lumbar Lordosis (PI-LL) angle mismatch on the 2D image of the standing position, to assign a spine alignment classification of: normal spine alignment (1), or flat back deformity alignment (2); and plan placement of an acetabular shell prosthesis based on the spinal mobility classification and the spine alignment classification.

In certain embodiments, the pre-operative 2D images are lateral views.

In certain embodiments, the spinal mobility classification of normal spine mobility (A) reflects a change in SS of at least 10° from the standing position to the seated position; and the spinal mobility classification of stiff spine mobility (B) reflects a change in SS of less than 10° from the standing position to the seated position.

In certain embodiments, the spinal alignment classification of normal spine alignment (1) reflects a PI-LL ± 10°, and the spinal alignment classification of flat back deformity alignment (2) reflects a PI-LL greater than (>) 10°.

In certain embodiments, the computer platform is operative to assign to the patient a combined classification based on the spinal mobility classification and the spine alignment classification, wherein the combined classification is selected from: 1A, 1B, 2A, and 2B.

In certain embodiments, the computer platform is operative to plan the placement by defining a default orientation of the acetabular shell prosthesis by an inclination angle of 40° and an anteversion angle of 20-25°, based on the patient having a combined classification of 1A or 2A.

In certain embodiments, the computer platform is operative to plan the placement by defining a default orientation of the acetabular shell prosthesis by an inclination angle of 45° and an anteversion angle of 25-30°, based on the patient having a combined classification of 1B.

In certain embodiments, the patient has a combined classification of 2B, and the computer platform is operative to evaluate a Posterior Pelvic Tilt on the 2D image of the standing position, to assign a posterior pelvic tilt classification of less than or equal to (≤) 13°, or greater than (>) 13°.

In certain embodiments, the computer platform is operative to plan the placement by defining a default orientation of the acetabular shell prosthesis by an inclination angle of 40° and an anteversion angle of 20-25°, based on the patient having the combined classification of 2B and the posterior pelvic tilt of less than or equal to (≤) 13°, or defining a default orientation of the acetabular shell prosthesis by an inclination angle of 45° and an anteversion angle of 25-30°, based on the patient having the combined classification of 2B and the posterior pelvic tilt of greater than (>) 13°.

In certain embodiments, the computer platform is operative to process the pre-operative 2D images of the patient by identifying bone landmarks on the pre-operative 2D images; analyzing the pre-operative 2D images; and displaying results of the analyzing on a display in communication with the computer platform.

In certain embodiments, the computer platform is further operative to process the pre-operative 2D images by identifying the bone landmarks automatically using a trained neural network, or receiving user input to manually identify the landmarks on the pre-operative 2D images.

In certain embodiments, the computer platform is further operative to identify the bone landmarks by receiving user input to manually confirm or correct a location of each of the bone landmarks.

In certain embodiments, the computer platform is further operative to analyze the pre-operative 2D images by drawing two or more anatomical reference lines based on the bone landmarks, and measuring angular measurements between the reference lines.

In certain embodiments, the computer platform is further operative to analyze the pre-operative 2D images by determining a spino-pelvic alignment parameter from the pre-operative 2D images.

In certain embodiments, the spino-pelvic parameter comprises a spino-pelvic tilt (SPT) angle, and the 2D images are lateral views of the patient in the standing position and the seated position.

In certain embodiments, the spino-pelvic parameter comprises a pelvic incidence (PI) angle, and the 2D images are lateral views of the patient in the standing position and the seated position.

In certain embodiments, the spino-pelvic parameter comprises a sacral slope (SS) angle, and the 2D images are lateral views of the patient in the standing position and the seated position.

In certain embodiments, the spino-pelvic parameter comprises an anterior pelvic plane tilt (APPT) angle, and the 2D images are lateral views of the patient in the standing position and the seated position.

In certain embodiments, the spino-pelvic parameter comprises pelvic incidence-lumbar lordosis (PI-LL) angle mismatch, and the 2D images are lateral views of the patient in the standing position and the seated position.

In certain embodiments, the computer platform is further operative to use the spino-pelvic alignment parameter as an input for planning the placement of the acetabular shell prosthesis.

These and other aspects, advantages and salient features of the invention will become apparent from the following detailed description, which, when taken in conjunction with the annexed drawings, where like parts are designated by like reference characters throughout the drawings, disclose embodiments of the invention.

### BRIEF DESCRIPTION OF THE DRAWINGS

Aspects of the present disclosure are illustrated by way of example and are not limited by the accompanying drawings. In the drawings:
**FIG.** 1 is an overhead view of a surgical system arranged during a surgical procedure in a surgical room which includes a camera tracking system for computer assisted navigation during surgery, and a surgical robot for robotic assistance according to some embodiments of the present disclosure.
**FIG. 2** illustrates the camera tracking system and the surgical robot of **FIG. 1****,** positioned relative to a patient according to some embodiments of the present disclosure.
**FIG. 3** further illustrates the camera tracking system and the surgical robot of **FIGS. 1-2****,** configured according to some embodiments of the present disclosure.
**FIG. 4** illustrates a block diagram of a surgical system that includes an extended reality headset, a computer platform, imaging devices, and a surgical robot, which are configured to operate according to some embodiments of the present disclosure.
**FIG. 5** illustrates a flowchart of a workflow during an intra-operative portion of a total hip arthroplasty (THA) surgery, in accordance with some embodiments of the present disclosure.
**FIG. 6** illustrates a flowchart of a patient preparation process before registration, in accordance with some embodiments of the present disclosure.
**FIG. 7** illustrates a radiographic inclination angle measured in the coronal plane of the patient, in accordance with some embodiments of the present disclosure.
**FIG. 8** illustrates a radiographic version angle measured relative to the coronal plane of the patient, in accordance with some embodiments of the present disclosure.
**FIG. 9** illustrates a workflow for planning the placement of an acetabular shell prosthesis based on the patient's spinal mobility classification and spine alignment classification, accordance with some embodiments of the present disclosure.
**FIGS. 10-11** illustrate a sub-workflow for processing pre-operative images of the patient used in the workflow of **FIG. 9****,** in accordance with some embodiments of the present disclosure.
**FIG. 12** illustrates a user interface for manually editing bone landmarks in a surgical planning application, in accordance with some embodiments of the present disclosure.
**FIG. 13** illustrates a user interface for automatically determining a spino-pelvic angle measurement in a surgical planning application, in accordance with some embodiments of the present disclosure.
**FIG. 14** illustrates the definition of the spino-pelvic tilt (SPT) angle, pelvic incidence (PI) angle, and sacral slope (SS) angles, parameters to be measured in accordance with some embodiments of the present disclosure.
**FIG. 15** illustrates the definition of the anterior pelvic plane tilt (APPT) angle, a parameter to be measured in accordance with some embodiments of the present disclosure.
**FIG. 16** illustrates the definition of the pelvic incidence-lumbar lordosis (PI-LL) angle mismatch, a parameter to be measured in accordance with some embodiments of the present disclosure.
**FIG. 17** illustrates a user interface for user-preferred spino-pelvic parameters in a surgical planning application, in accordance with some embodiments of the present disclosure.

It is noted that the drawings of the disclosure are not necessarily to scale. The drawings are intended to depict only typical aspects of the disclosure, and therefore should not be considered as limiting the scope of the disclosure. In the drawings, like numbering represents like elements between the drawings.

### DETAILED DESCRIPTION

As noted, the present disclosure relates to devices, systems, and methods for use in planning and performing navigated surgical procedures including total hip arthroplasty (THA) procedures. In particular, the disclosure provides a framework for pre-operatively planning the placement of an acetabular shell prosthesis, considering spino-pelvic alignment, based on parameters extracted from pre-operative 2D images, e.g., x-ray images of the patient. This planning framework may be used in a THA procedure planned and performed using a surgical system as described herein to mitigate the risk of post-operative hip instability, dislocation, and revision.

The present application is related to (1) US Patent Application No. 15/180,126, filed June 13, 2016 (U.S. Pat. 10,842,453), (2) US Patent Application No. 15/157,444, filed May 18, 2016 (U.S. Pub. No. 2016/0256225), (3) US Patent Application No. 18/743,685, filed June 14, 2024, (4) US Patent Application No. 18/743,388, filed June 14, 2024, (5) US Patent Application No. 18/743,647, filed June 14, 2024, (6) US Patent Application No. 18/743,615, filed June 14, 2024, (7) US Patent Application No. 18/737,123, filed June 07, 2024, (8) US Patent Application No. 18/770,993, filed July 12, 2024, (9) US Patent Application No. 18/802,689, filed August 13, 2024, (10) US Patent Application No. 18/801,924, filed August 13, 2024, (11) US Patent Application No. 18/903,032, filed October 1, 2024 (Attorney Docket No. IDR-23-152 / COXA-IDR-07), and (12) US Patent Application No. 18/909,473, filed October 8, 2024 (Attorney Docket No. IDR-24-050), each of which is incorporated herein by reference.

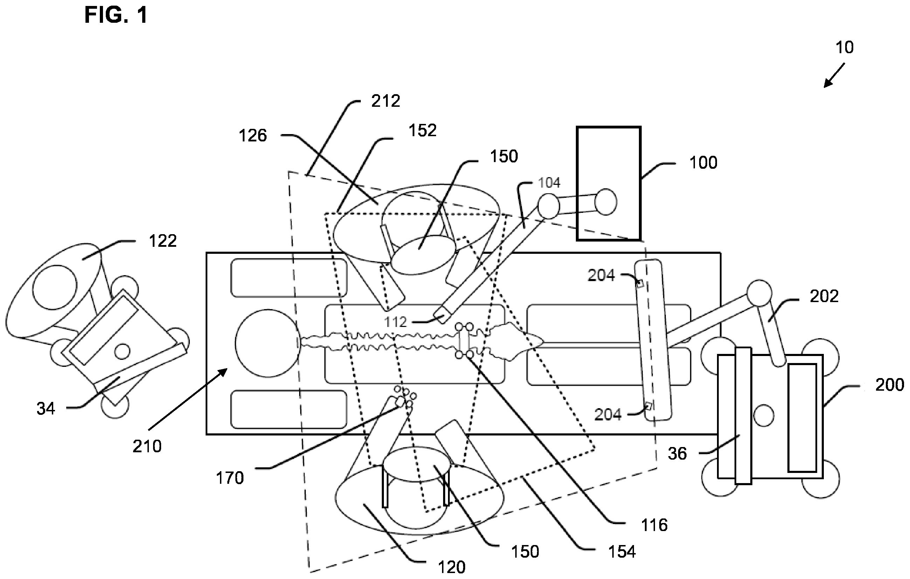

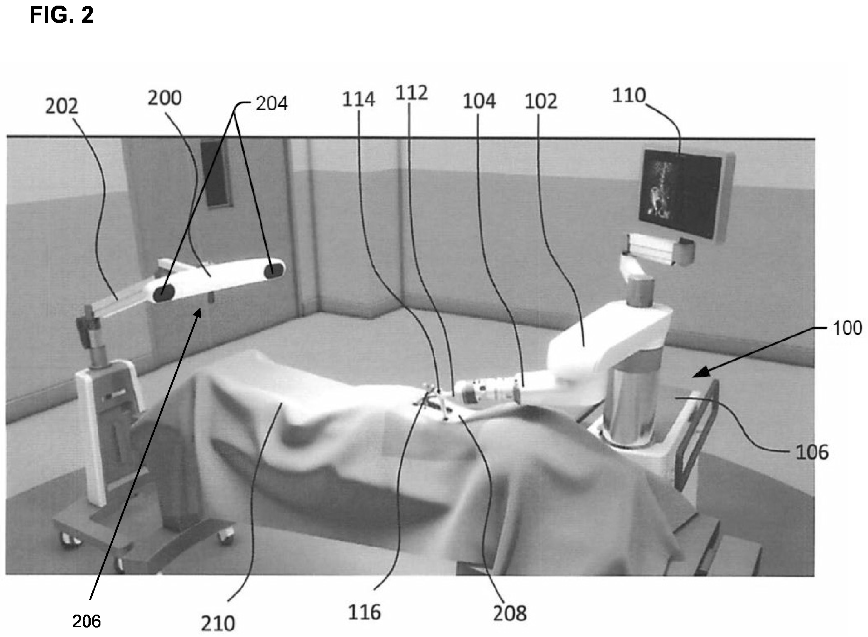

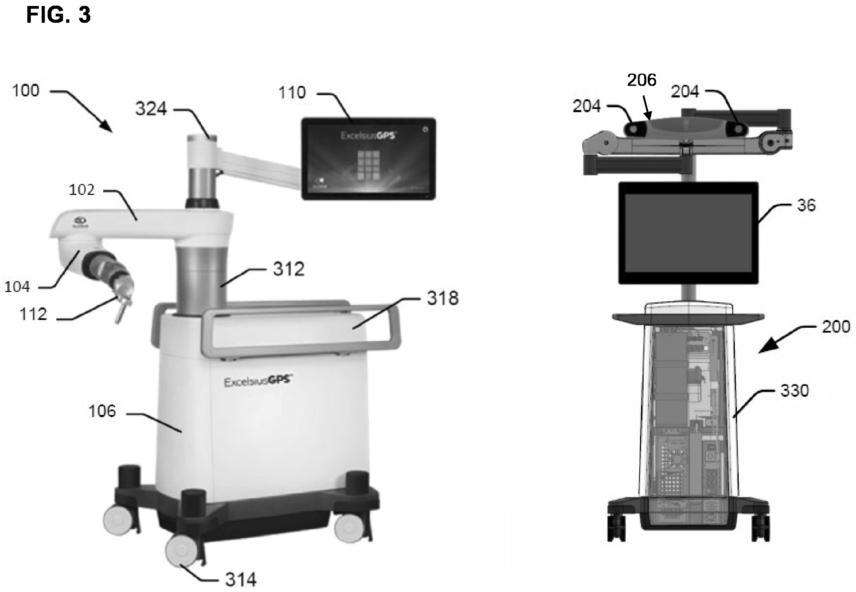

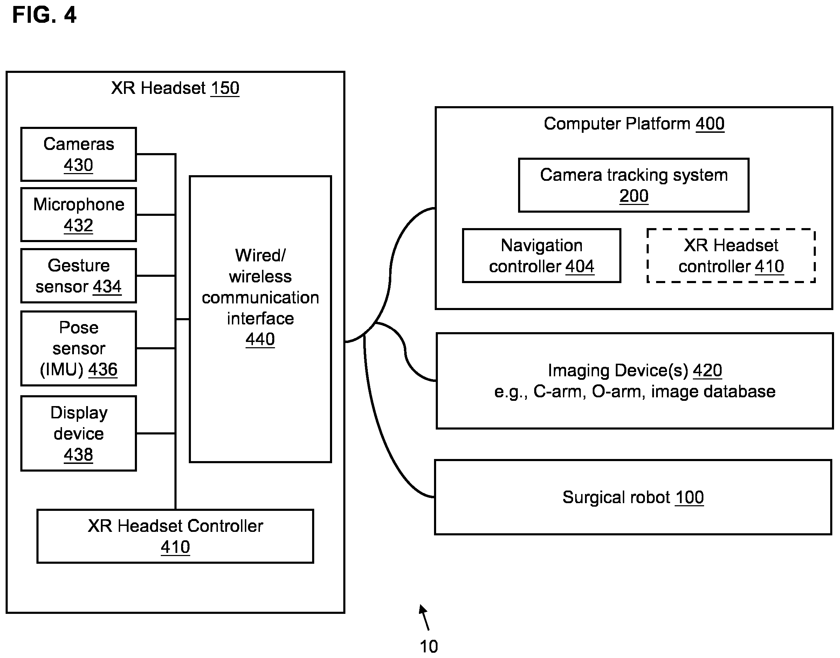

Turning to the figures, **FIG. 1** is an overhead view of a surgical system 10 arranged during a surgical procedure in a surgical or operating room. The system 10 includes a camera tracking system 200 for computer assisted navigation during surgery and may further include a surgical robot 100 for robotic assistance according to some embodiments. **FIG. 2** illustrates the camera tracking system 200 and the surgical robot 100 positioned relative to a patient according to some embodiments. **FIG. 3** further illustrates the camera tracking system 200 and the surgical robot 100 configured according to some embodiments. **FIG. 4** illustrates a block diagram of a surgical system 10 that includes an extended reality (XR) headset 150, a computer platform 400, imaging devices 420, and the surgical robot 100 which are configured to operate according to some embodiments.

The camera tracking system 200 **(****FIGS. 1-4****)** in some cases includes an intraoperative imaging system, that can include distinct imaging modalities, e.g., one or more of fluoroscopy, 2D Radiography, and cone-beam computed tomography (CBCT). The camera tracking system 200 is capable of: (1) capturing 3-Dimensional (3D) images (e.g., CT, CBCT, MCT, PET, Angiogram, MRI, ultrasound, etc.), (2) capturing 2-Dimensional (2D) images (e.g., fluoroscopy, digital radiography, ultrasound, etc.), and (3) containing an integrated or detachable navigation array having tracking markers (e.g., NIR retroreflective, NIR LED, visible, etc.), which is calibrated to the image space of the 2D and 3D images.

The surgical robot 100 is capable of: (1) using registered 2D and/or 3D images for surgical planning, navigation, and guidance in a variety of workflows (e.g., intraoperative 3D, intraoperative 2D, preoperative 3D to 2D, and intraoperative 3D to 2D, etc.); and (2) containing a camera tracking system 200 capable of tracking markers (e.g., NIR retroreflective, NIR LED, visible, etc.). In some cases, as noted herein, a dynamic reference base (DRB) (or patient reference array) 116 is (1) capable of rigidly attaching to the patient anatomy, and (2) contains an array of tracking markers (e.g., NIR retroreflective, NIR LED, visible, etc.).

The XR headsets 150 may be configured to augment a real-world scene with computer generated XR images while worn by personnel in the operating room. The XR headsets 150 may be configured to provide an augmented reality (AR) viewing environment by displaying the computer generated XR images on a see-through display screen that allows light from the real-world scene to pass therethrough for combined viewing by the user. Alternatively, the XR headsets 150 may be configured to provide a virtual reality (VR) viewing environment by preventing or substantially preventing light from the real-world scene from being directly viewed by the user while the user is viewing the computer-generated AR images on a display screen. The XR headsets 150 can be configured to provide both AR and VR viewing environments. Thus, the term XR headset encompasses both or either of an AR headset or a VR headset.

With continuing reference to **FIGS. 1-4****,** the surgical robot 100 may include, for example, one or more robot arms 102, 104, a display 110, an end effector 112 including, e.g., a guide tube and an end effector reference element 114 which can include one or more tracking fiducials. A patient reference array (or DRB) 116 (shown in **FIG. 1****)** has a plurality of tracking fiducials and is secured directly to the patient 210. For example, a navigated pelvis DRB marker array may be placed intra-incision or extra-incision with the help of cortical pins drilled into the pelvic bone. In some embodiments, the DRB is oriented to be visible by the tracking camera(s) 204 (e.g., a stereoscopic tracking camera) of camera tracking system 200 and/or the XR headset 150. A reference element 170 is attached to or formed on an instrument, surgical tool, surgical implant device, etc.

The camera tracking system 200 includes tracking cameras 204 which may be spaced apart to provide stereo cameras configured with partially overlapping fields-of-view. The camera tracking system 200 can have any suitable configuration of arm(s) 202 to move, orient, and support the tracking cameras 204 in a desired location, and may contain at least one processor operable to track the location of an individual fiducial and pose of an array of fiducials of a reference element. As used herein, the term "pose" refers to the location (e.g., along three orthogonal axes, e.g., the x-, y-, and z-axes) and/or the rotation angle (e.g., about the three orthogonal axes) of fiducials (e.g., DRB) relative to another fiducial (e.g., surveillance fiducial) and/or to a defined coordinate system (e.g., camera coordinate system, navigation coordinate system, etc.).

The tracking cameras 204 may include, e.g., infrared cameras (e.g., bifocal or stereophotogrammetric cameras) operable to identify, for example, active and passive tracking fiducials for single fiducials (e.g., a surveillance fiducial) and reference elements which can be formed on or attached to the patient 210 (e.g., patient reference element or DRB 116), end effector 112 (e.g., end effector reference element 114), XR headset(s) 150 worn by a surgeon 120 and/or a surgical assistant 126, etc. in a given measurement volume of a camera coordinate system while viewable from the perspective of the tracking cameras 204. The tracking cameras 204 may scan the given measurement volume and detect light that is emitted or reflected from the fiducials in order to identify and determine locations of individual fiducials and poses of the reference elements in three-dimensions. For example, active reference elements may include infrared-emitting fiducials that are activated by an electrical signal (e.g., infrared light emitting diodes (LEDs)), and passive reference elements may include retroreflective fiducials that reflect infrared light (e.g., they reflect incoming IR radiation into the direction of the incoming light), for example, emitted by illuminators on the tracking cameras 204 or other suitable device.

The XR headsets 150 may each include tracking cameras (e.g., spaced apart stereo cameras) that can track the location of a surveillance fiducial and poses of reference elements within the XR camera headset fields of view (FOVs) 152 and 154, respectively. Accordingly, as illustrated in **FIG. 1****,** the location of the surveillance fiducial and the poses of reference elements on various objects such as, e.g., instrument reference element 170 and patient reference element 116, can be tracked while in the FOVs 152 and 154 of the XR headsets 150 and/or a FOV 212 of the tracking cameras 204.

**FIGS. 1** **and** **2** illustrate a potential configuration for the placement of the camera tracking system 200 and the surgical robot 100 in an operating room environment. Computer assisted navigated robotic surgery can be provided by the surgical robot 100, the camera tracking system 200 controlling the XR headsets 150 and/or other displays 34, 36, and 110 to display surgical procedure navigation information.

The camera tracking system 200 may operate using tracking information and other information provided by multiple XR headsets 150 such as inertial tracking information and optical tracking information (frames of tracking data). The XR headsets 150 operate to display visual information and may playout audio information to the wearer. This information can be from local sources (e.g., the surgical robot 100), imaging devices 420 **(****FIG. 4****),** remote sources (e.g., patient medical image database), and/or other electronic equipment. The camera tracking system 200 may track fiducials in 6 degrees-of-freedom (6 DOF) relative to three axes of a 3D coordinate system and rotational angles about each axis. The XR headsets 150 may also operate to track hand poses and gestures to enable gesture-based interactions with "virtual" buttons and interfaces displayed through the XR headsets 150, and can also interpret hand or finger pointing or gesturing as various defined commands.

An "outside-in" machine vision navigation bar 206 supports the tracking cameras 204 and may include a color camera. The machine vision navigation bar generally has a more stable view of the environment because it does not move as often or as quickly as the XR headsets 150 while positioned on wearers' heads. The patient reference element (or, DRB) 116 is generally rigidly attached to the patient 210 with stable pitch and roll relative to gravity. This local rigid patient reference 116 can serve as a common reference for reference frames relative to other tracked elements, such as a reference element 114 on the end effector 112, instrument reference element 170, and reference elements on the XR headsets 150.

In some embodiments, at the end of the end effector 112, instruments are connected to perform operations such as resection, reaming, and implant placement.

The surgical robot 100 may be positioned near or next to patient 210 as shown in **FIGS. 1-2****.** The robot 100 can be positioned at any suitable location near the patient 210 depending on the area of the patient 210 undergoing the surgical procedure. The camera tracking system 200 may be separate from the robot system 100 and positioned at the foot of patient 210. This location allows the tracking camera 200 to have a direct visual line of sight to the surgical area 208, e.g., the hip area **(****FIG. 2****).** In the configuration shown in **FIG. 1****,** the surgeon 120 may be positioned across from the robot 100, but is still able to manipulate the end effector 112 and the display 110. A surgical assistant 126 may be positioned across from the surgeon 120 again with access to both the end effector 112 and the display 110. If desired, the locations of the surgeon 120 and the assistant 126 may be reversed. An anesthesiologist 122, nurse, or scrub tech can operate equipment which may be connected to display information from the camera tracking system 200 on a display 34 **(****FIG. 1****).**

With respect to the other components of the robot 100, the display 110 can be attached to the surgical robot 100 or in a remote location. The end-effector 112 may be coupled to the robot arm 104 and be controlled by at least one motor. An upper arm 102 may further couple the arm 104 to the column 312 of the robot 100. In some embodiments, end effector 112 includes a guide tube, which is configured to receive and orient a surgical instrument, tool, or implant used to perform a surgical procedure on the patient 210. For example, the end effector 112 is adapted to receive a surgical instrument or a portion thereof, to removably couple to the instrument, and to manipulate the instrument such as by translating and rotating the instrument. In some other embodiments, the end-effector 112 includes a passive structure guiding a saw blade (e.g., sagittal saw) along a defined cutting plane. Although generally shown with a guide tube, it will be appreciated that the end-effector 112 may be replaced with any suitable instrumentation for use in surgery.

The surgical robot 100 is operable to control the translation and orientation of the end effector 112. The robot 100 may move the end-effector 112 under the control of the computer platform, e.g., along x-, y-, and z-axes. The end-effector 112 can be configured for selective rotation about one or more of the x-, y-, and z-axes, and a Z Frame axis, such that one or more of the Euler Angles (e.g., roll, pitch, and/or yaw) associated with the end effector 112 can be selectively computer controlled. In some embodiments, selective control of the translation and orientation of end effector 112 and associated surgical instrument can permit performance of medical procedures with significantly improved accuracy compared to conventional robots that utilize, for example, a six-DOF robot arm comprising only rotational axes. For example, the surgical robot 100 may be used to operate on patient 210, and robot arm 104 can be positioned above the body of patient 210, with end-effector 112 selectively angled relative to the z-axis toward the body of patient 210.

In some example embodiments, the XR headset(s) 150 can be controlled to dynamically display an updated graphical indication of the pose of the surgical instrument so that the user, e.g., surgeon 120, can be aware of the pose of the surgical instrument at all times during the procedure.

Fiducials of reference elements can be formed on or connected to robot arms 102 and/or 104, the end effector 112 (e.g., end effector element 114 in **FIG. 2****),** and/or a surgical instrument (e.g., instrument element 170) to enable tracking of poses in a defined coordinate system, e.g., such as in six degrees of freedom (DOF) along three orthogonal axes and rotation about the axes. The reference elements 114, 116, 170 enable each of the marked objects (e.g., the end-effector 112, the patient 210, and the surgical instruments, respectively) to be tracked by the tracking camera 200, and the tracked poses can be used to provide navigated guidance during a surgical procedure and/or to control movement of the surgical robot 100 for guiding the end effector 112 and/or an instrument manipulated by the end effector 112. The instrument manipulated by the end effector 112 may include, e.g., a reamer 124 or an inserter adapted to insert an implant.

Referring to **FIG. 3****,** the surgical robot 100 may include a display 110, upper arm 102, lower arm 104, end effector 112, vertical column 312, casters 314, a table 318, and ring 324 which uses lights to indicate statuses and other information. Cabinet 106 may house electrical components of surgical robot 100 including, but not limited to, a battery, a power distribution module, a platform interface board module, and a computer. The camera tracking system 200 may include a display 36, tracking cameras 204, arm(s) 202 **(****FIG. 1****),** a computer housed in cabinet 330, and other components.

In computer assisted navigated surgeries, perpendicular 2D scan slices, such as axial, sagittal, and/or coronal views of patient anatomical structure are displayed to enable user visualization of the patient's anatomy alongside the relative poses of surgical instruments. An XR headset or other display can be controlled to display one or more 2D scan slices of patient anatomy along with a 3D graphical model of anatomy. The 3D graphical model may be generated from a 3D scan of the patient, e.g., by a CT scan device, and/or may be generated based on a baseline model of anatomy which isn't necessarily formed from a scan of the patient.

### Example Surgical System

**FIG. 4** illustrates a block diagram of a surgical system 10 that includes a surgical robot 100, a computer platform 400 including, *inter alia,* the camera tracking system 200, imaging device(s) 420, and XR headset(s) 150 which are configured to operate as described herein to plan and execute a surgical procedure such as a THA, according to some embodiments.

The imaging device(s) 420 may include a C-arm imaging device, an O-arm imaging device, another imaging device, and/or a patient image database of 2D and/or 3D images, which may be DICOM-formatted images. The XR headset 150 provides a human interface for performing navigated surgical procedures. The XR headset 150 can be configured to provide functionalities, e.g., via the computer platform 400, that include without limitation any one or more of: identification of hand gesture-based commands, and display of XR graphical objects on a display device 438 of the XR headset 150 and/or another display device. The display device 438 may include a video projector, flat panel display, etc. The user may view the XR graphical objects as an overlay anchored to particular real-world objects viewed through a see-through display screen. The XR headset 150 may additionally or alternatively be configured to display on the display device 438 video streams from cameras mounted to one or more XR headsets 150 and other cameras.

Electrical components of the XR headset 150 can include a plurality of cameras 430, a microphone 432, a gesture sensor 434, a pose sensor (e.g., inertial measurement unit (IMU)) 436, the display device 438, and a wireless/wired communication interface 440. The cameras 430 of the XR headset 150 may be visible light capturing cameras, near infrared capturing cameras, or a combination of both.

The cameras 430 may be configured to operate as the gesture sensor 434 by tracking for identification user hand gestures performed within the field-of-view of the camera(s) 430. Alternatively, the gesture sensor 434 may be a proximity sensor and/or a touch sensor that senses hand gestures performed proximately to the gesture sensor 434 and/or senses physical contact, e.g., tapping on the sensor 434 or its enclosure. The pose sensor 436, e.g., IMU, may include a multi-axis accelerometer, a tilt sensor, and/or another sensor that can sense rotation and/or acceleration of the XR headset 150 along one or more defined coordinate axes. Some or all of these electrical components may be contained in a head-worn component enclosure or may be contained in another enclosure configured to be worn elsewhere, such as on the hip or shoulder.

As explained above, the surgical system 10 includes the camera tracking system 200 which may be connected to a computer platform 400 for operational processing and which may provide other operational functionality including a navigation controller 404 and/or an XR headset controller 410. The surgical system 10 may further include the surgical robot 100. The navigation controller 404 can be configured to provide visual navigation guidance to an operator for moving and positioning a surgical tool relative to patient anatomical structure based on a surgical plan, e.g., from a surgical planning function, defining where a surgical procedure is to be performed using the surgical tool on the anatomical structure and based on a pose of the anatomical structure determined by the camera tracking system 200. The navigation controller 404 may be further configured to generate navigation information based on a target pose for a surgical tool, a pose of the anatomical structure, and a pose of the surgical tool and/or an end effector 112 of the surgical robot 100. The navigation information may be displayed through the display device 438 of the XR headset 150 and/or another display device to indicate where the surgical tool and/or the end effector 112 of the surgical robot 100 should be moved to perform a surgical procedure according to a defined surgical plan.

The electrical components of the XR headset 150 can be operatively connected to the electrical components of the computer platform 400 through the wired/wireless interface 440. The electrical components of the XR headset 150 may be operatively connected, e.g., through the computer platform 400 or directly connected, to various imaging devices 420, e.g., the C-arm imaging device, the O-arm imaging device, another imaging device(s), the patient image database, and/or to other medical equipment through the wired/wireless interface 440.

The surgical system 10 may include a XR headset controller 410 that at least partially resides in the XR headset 150, the computer platform 400, and/or another system component connected via wired cables and/or wireless communication links. Various functionality may be provided by software executed by the XR headset controller 410. The XR headset controller 410 is configured to receive information from the camera tracking system 200 and the navigation controller 404, and to generate an XR image based on the information for display on the display device 438.

The XR headset controller 410 can be configured to operationally process frames of tracking data from the cameras 430 (tracking cameras), signals from the microphone 432, and/or information from the pose sensor 436 and the gesture sensor 434, to generate information for display as XR images on the display device 438 and/or for display on other display devices for user viewing. Thus, the XR headset controller 410 as illustrated as a circuit block within the XR headset 150 is to be understood as being operationally connected to other illustrated components of the XR headset 150 but not necessarily residing within a common housing or being otherwise transportable by the user. For example, the XR headset controller 410 may additionally or alternatively reside within the computer platform 400 which, in turn, may reside within the cabinet 330 of the camera tracking system 200, the cabinet 106 of the surgical robot 100, etc.

### Exemplary Patient Registration Workflows

In some embodiments of the present disclosure, the system 10, e.g., computer platform 400, may perform one of a number of available workflows to register a patient to the surgical system 10 prior to surgery. The workflows may further include isolating a target area for the surgical procedure from non-target surgical areas. In one example, the target surgical area may include the acetabulum, and the non-target surgical area may include the femur.

In various embodiments, the workflow may be image-based or may be imageless, in which no pre-operative images are used. In the latter case, information about the patient anatomy in the operating room (OR) can be obtained by the surgeon measuring key parameters of the patient's bone(s) using the system as described herein. For example, the computer platform 400 of the system 10 operates to identify the locations of landmarks (e.g., points, axes, and/or surfaces) on the bone and to register the locations either concurrently with the identification or thereafter. The locations can be used to define reference plane(s) such as, e.g., the anterior pelvic plane (APP) and/or functional pelvic plane (FPP)) which, in turn, are used to plan implants and navigate the robot and surgical instruments for THA surgical procedures.

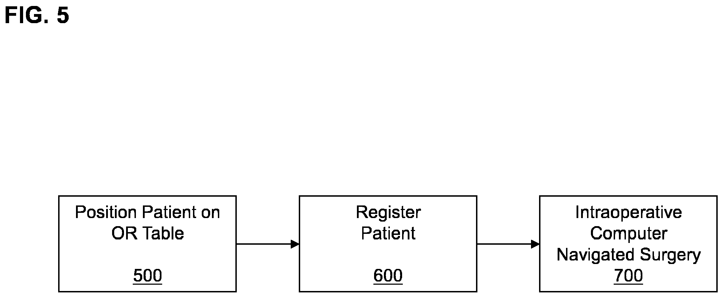

**FIG. 5** illustrates a flowchart for an imageless workflow during an intra-operative portion of a THA surgery, in accordance with some embodiments of the present disclosure. In some embodiments, after positioning the patient on the operating room table (process 500), some of the operations discussed above and below may be performed during process 600 to register a patient and before another process 700 for intraoperative computer navigated surgery. In the case of a hip, a pelvis or acetabulum of the patient is registered in the tracking coordinate system of the camera tracking system 200. As shown in **FIG. 1****,** the pelvis or more particularly the acetabulum is registered in the optical coordinate system. In one embodiment, the registration is done in an imageless modality without the use of any medical images such as X-rays or CT images from an imaging device. As noted herein, in other embodiments, registration is performed using one or more preoperative X-ray images and/or CT images.

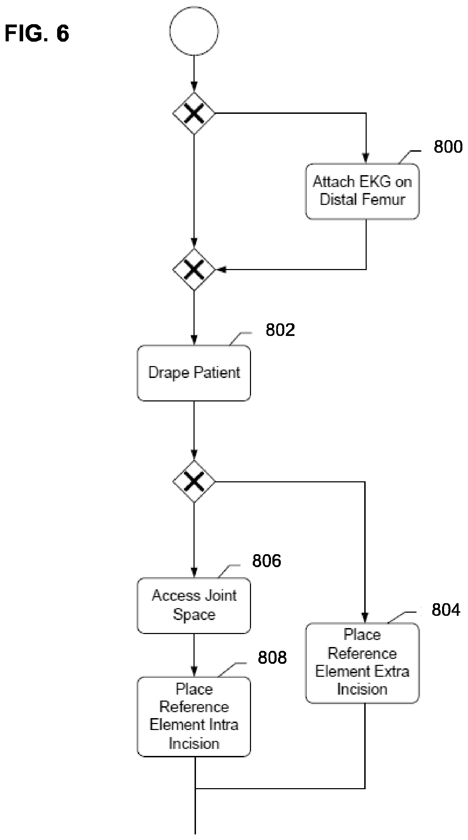

**FIG. 6** illustrates a flowchart of a patient preparation process before registration, in accordance with some embodiments of the present disclosure.

The patient preparation process may begin with a patient being positioned in a lateral or supine position on the OR table. The patient's body is prepared for registration. Optionally, in process 800, an EKG/ECG patch electrode is attached on or adjacent a distal end of the patient's femur. The EKG/ECG patch electrode may be placed on the center of the patella or slightly inferior to the center. In some embodiments, the patch location is in line with the anatomic axis of the femur. This patch may be used to acquire the most distal point of the femur under the drape at a later stage. This patch may also be used to track the femur in space (e.g., when the patient's leg is moved during surgery) and may also be used to assist in measuring the patient's leg length.

In process 802, the patient body is draped. Then, depending on the surgeon's technique, the navigated pelvis DRB, e.g., DRB 116 **(****FIG. 2****),** is placed intra-incision (processes 806-808) or extra-incision (process 804) with the help of cortical pins drilled into the pelvic bone. In some embodiments, the DRB 116 is oriented to be visible to the tracking camera(s), e.g., stereoscopic tracking cameras 204 installed on the camera tracking system 200 **(****FIG. 1****)** or the XR headset 150 **(****FIG. 1****).** In one embodiment, the operation to place the DRB intra-incision includes using the system 10 to track and navigate access to the joint space (process 806) and placing the reference element intra-incision. In an alternative embodiment, the reference element 116 is placed extra-incision (process 804) and the system does not necessarily need to be used to track and navigate access to the joint space.

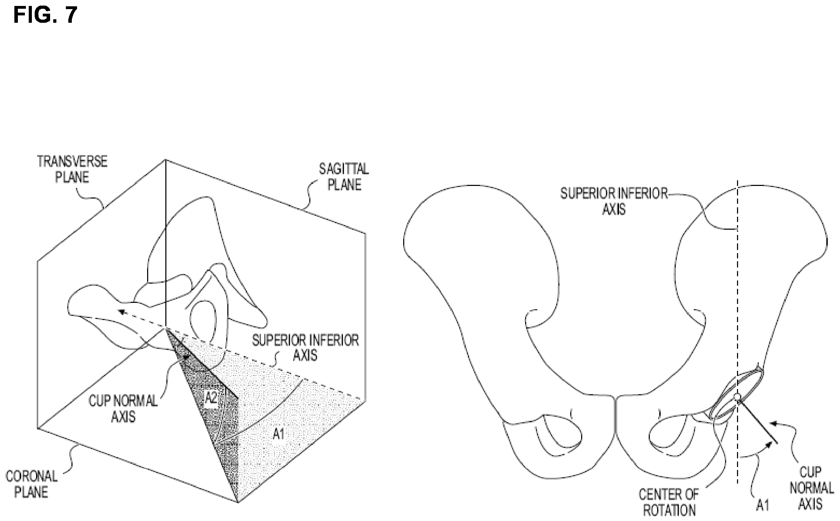

After the reference element, e.g. DRB 116 has been placed intra-incision or extra-incision, data points and axes can be collected on the patient anatomy with the assistance of navigated instruments and using the pelvis DRB coordinate system as a spatial reference. In addition to this, two pelvic reference planes can be established to plan placement of implants by measuring angular deviations such as inclination and version of the acetabular cup implant as shown in **FIGS. 7-8****.**

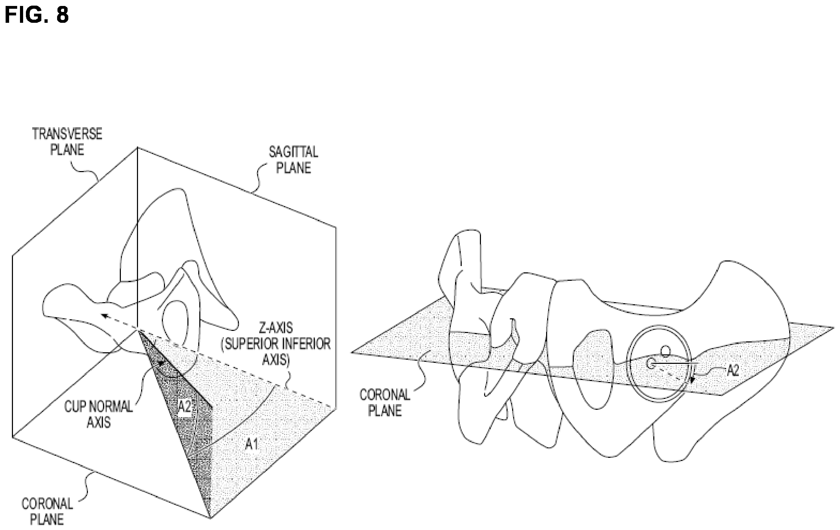

**FIG. 7** illustrates a radiographic inclination angle (A1) measured in the coronal plane of the patient, in accordance with some embodiments of the present disclosure. In some embodiments, the surgeon may use a navigated instrument to palpate or paint the surface of the acetabular cavity of the patient to determine a center of rotation of the acetabulum. **FIG. 8** illustrates a radiographic version angle (A2) measured relative to the coronal plane of the patient, in accordance with some embodiments of the present disclosure.

It is to be understood herein that although the user interfaces and associated operations are described as being performed in a certain sequence, they may be performed in other sequences while still being within disclosed embodiments. Moreover, it is not necessary that all of the user interfaces and/or described operations be performed. Instead, fewer operations may be performed while still being within disclosed embodiments. Further, additional registration approaches can include image-based and imageless workflows. Combinations of these registration approaches are also possible in keeping with the various disclosed embodiments.

US Patent Application No. 18/430,077 further discloses processes for registration of a pelvic acetabulum of a patient (including painting the acetabular cavity), in accordance with various embodiments of the present disclosure. For example, to define the APP and FPP origins, the pelvic acetabular center of rotation can be determined after removing the femoral head of the patient from the acetabular cavity. The acetabular cavity may be made accessible by cutting the femoral neck and by removing the femoral head from the acetabular cavity. In some embodiments, a cork screw instrument may be used to remove the femoral head from the acetabular cavity.

The surface of the acetabular cavity can then be painted using the navigated instrument (e.g., a stylus). For example, the surgeon may use the navigated instrument (e.g., stylus) to palpate the surface of the acetabular cavity, as the tracking camera measures the position of a ball on the end of the stylus in a continuous way. This process provides a cloud of points for the measured positions (locations). At the same time, the tracking camera may also monitor and track the pose of the patient DRB 116 attached to the pelvis such that the pose of the stylus can be tracked relative to the pose of the patient DRB. Alternatively, the surgeon may subsequently measure a predefined number or percentage of points by palpating them one-by-one. Based on these points and the tracking data of the stylus and patient DRB 116, the center of rotation of the pelvic acetabular cavity is determined. Additionally, based on these points, the surface of the acetabular cavity may be registered in the system and/or a 3D model may be generated or modified based on these points. Next, the acetabular cavity shape can be recreated (e.g., in a 3D model) by the system based on the measured cloud of points and using other algorithms, e.g., for outlier removals and surface fitting.

While certain imageless approaches are described herein and in US Patent Application No. 18/430,077 (filed February 01, 2024), previously incorporated by reference herein, other example methods of performing imageless and image-based registration of the pelvis to the tracking coordinate system of the tracking system (e.g. optical coordinate system). These methods may also be used to, e.g., determine a native center of rotation of the acetabulum, derive or define an FPP, and derive or define an APP. Registration may allow a navigation system or robotic system to track any navigated instrument or end effector 112 or any tool attached to the end effector 112 relative to the pelvis as tracked by a patient dynamic reference base 116 attached to the pelvis. Various registration methods described herein can be combined in keeping with various disclosed embodiments.

For example, in one imageless method, an APP is derived by either touching various known points (e.g., left and right anterior superior iliac spine (ASIS) and pubic symphysis) with a navigated instrument, or by a physician lining up a plane or axis defined by the navigated instrument along or parallel to the APP. With the center of rotation and APP determined, the system (either a navigation system or a combined navigation and robot system 100) has sufficient information to register the acetabulum in the coordinate system (e.g., optical coordinate system) of the camera tracking system 200. In both of the above-noted example methods, the tracking system may be constantly monitoring and tracking the pose of the patient DRB 116 attached to the pelvis while also tracking the navigated instrument (e.g., stylus) such that the pose of the instrument can be tracked relative to the pose of the patient DRB, at least for purposes of registering the pelvis relative to the patient DRB 116 in the tracking coordinate system of the camera tracking system 200.

Some exemplary image-based examples include the use of preoperative CT images. In one such exemplary image-based approach to patient registration, pre-operative CT and intra-operative fluoroscopy images are merged, the non-target surgical area is excluded, and the location of the target surgical area is registered based on the merged CT image and fluoroscopy image, as described in Patent Application No. 18/743,388 (filed June 14, 2024). In another exemplary image-based approach to patient registration, pre-operative CT and intra-operative point cloud data acquired via a navigated instrument are merged, and the location of the target surgical area is registered based on the merged CT image and point cloud data, as described in Patent Application No. 18/743,615 (filed June 14, 2024).

Other exemplary image-based registration approaches can be performed without first obtaining pre-operative CT images. In one such exemplary approach to patient registration, intra-operative fluoroscopy images are obtained, and an APP and FPP are identified. The FPP images and APP images are merged, excluding the non-target surgical area, and the location of the target surgical area is registered based on the merged APP and FPP fluoroscopy images, as described in Patent Application No. 18/743,647 (filed June 14, 2024).

In a further exemplary image-based approach to patient registration, intra-operative fluoroscopy images are obtained, and intra-operative point cloud data is acquired using a navigated instrument. An FPP is identified in the intra-operative fluoroscopy images, and inputs from a navigated instrument about a location of the target surgical area are obtained. A set of landmarks relative to the identified FPP are verified using the inputs from the navigated instrument, and the location of the target surgical area is registered based on the identified FPP images and inputs from the navigated instrument, as described in Patent Application No. 18/743,685 (filed June 14, 2024).

### Spino-pelvic planning from 2D images

The surgical system 10 including computer platform 400 as described above may be used to plan and subsequently perform THA procedures, including planning placement of the acetabular shell prosthesis (or cup) component. Such planning incorporates a determination of an appropriate position and orientation in a 3D space of the prosthesis inside the operated pelvis acetabulum. The acetabular shell orientation is defined by two types of rotation, namely, inclination **(****FIG. 7****)** and (ante)version **(****FIG. 8****),** described above. These rotations are referenced with respect to the patient coronal and sagittal body planes.

Planning and performance of a THA procedure includes selecting patient-specific acetabular shell inclination and version angles to provide the patient with optimal leg mobility, and to minimize dislocation risks between the implanted pelvic acetabular shell and femoral head. Surgeons typically use "safe zones" which are discussed extensively in medical literature, and define acceptable angular ranges for the inclination and version of the acetabular shell. For example, the Lewinnek safe zone defines the acetabular inclination to be within 40 ± 10°, and the acetabular version to be within 15 ± 10° in order to minimize dislocation risk.

Patients with lumbar fusion, spinal deformity, and/or abnormal spino-pelvic mobility present a higher risk for instability, dislocation, and revision after THAs. For such patients, the use of standardized "safe zones," which are agnostic from inherent patient spine abnormalities, may produce sub-optimal outcomes. An optimal acetabular shell placement for a patient with spinal deformities takes into consideration spino-pelvic alignment parameters and bone mobility compensation mechanisms that lead to abnormal spino-pelvic motion.

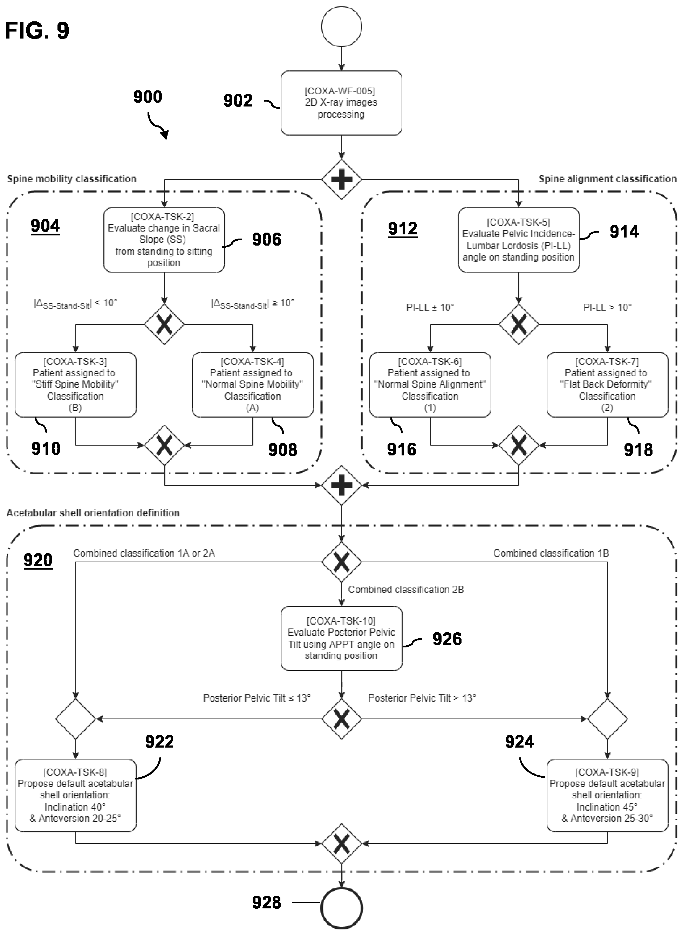

Turning to **FIG. 9****,** a workflow 900 is provided for planning acetabular shell placement, including optimal acetabular shell orientation, based on spino-pelvic alignment. Such planning may be performed using computer platform 400 of the surgical system 10.

The workflow 900 begins with obtaining pre-operative images of the patient in each of a standing position and a seated position (not shown). These pre-operative images may be 2D images, e.g., x-ray images, and may be taken along a lateral view. These images may be used as input for the workflow 900.

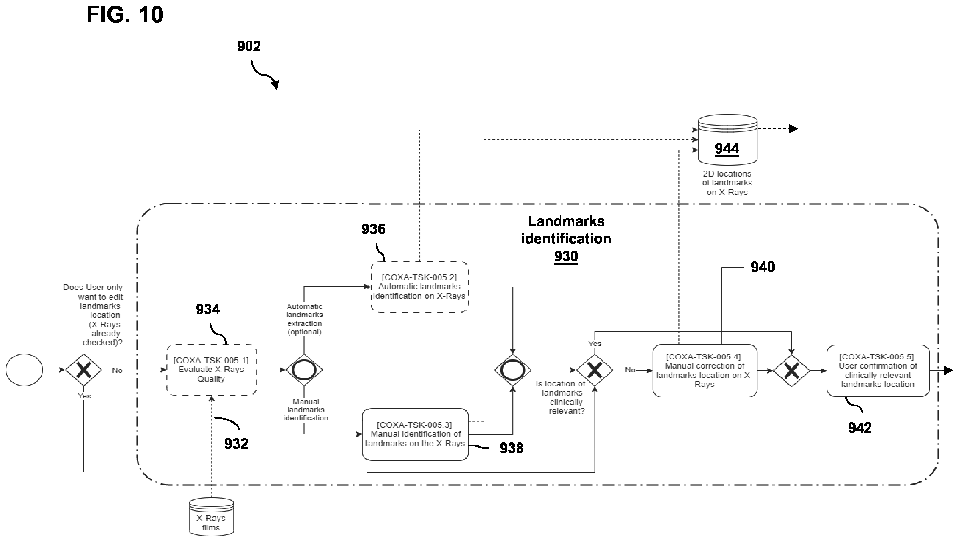

Process 902 includes processing the 2D images for use in the subsequent workflow processes, e.g., processes 904, 912. Image processing may include a number of subprocesses which are described in greater detail below and in reference to **FIGS. 10-11****,** which collectively yield the patient-specific spino-pelvic alignment parameters described herein. These patient-specific spino-pelvic alignment and parameters are used to identify a patient's pathology class.

Returning to **FIG. 9****,** process 904 classifies the patient's spine mobility, according to subprocesses 906, 908, and 910. Subprocess 906 evaluates a change in sacral slope (SS) angle in the 2D images from the standing position to the seated position. This is computed by comparing the patient's SS angle as determined in the 2D image of the patient in the standing position, with the patient's SS angle as determined in the 2D image of the patient in the seated position. Based on the SS angle change evaluation at subprocess 904, the patient's spinal mobility may be classified. In particular, subprocess 908 assigns a spinal mobility classification of "normal spine mobility" ("A") where the change in SS from standing to seated position is greater than or equal to 10°, which can be expressed as: | Δ_{SS-Stand-Sit} | ≥ 10°.

Subprocess 910 assigns a spinal mobility classification of "stiff spine mobility" ("B") where the change in SS from standing to seated position is less than (<) 10°, which can be expressed as: | Δ_{SS-Stand-Sit} | < 10°.

Process 912 classifies the patient's spinal alignment, according to subprocesses 914, 916, and 918. Process 912 may be executed in parallel with process 904, although series execution may also performed.

Subprocess 914 evaluates the patient's Pelvic Incidence-Lumbar Lordosis (PI-LL) angle mismatch on a 2D image taken in the standing position. Based on the patient's PI-LL angle mismatch, the patient's spinal alignment may be classified. In particular, subprocess 916 assigns a spinal alignment classification of "normal spine alignment" ("1") where the patient's PI-LL mismatch ranges from -10° to +10° (PI-LL ± 10°). Subprocess 918 assigns a spinal alignment classification of "flat back deformity" ("2") where the patient's PI-LL mismatch is greater than 10° (PI-LL > 10°). Patients with flatback deformity have straightening of the normal curves in the lower spine (lumbar lordosis).

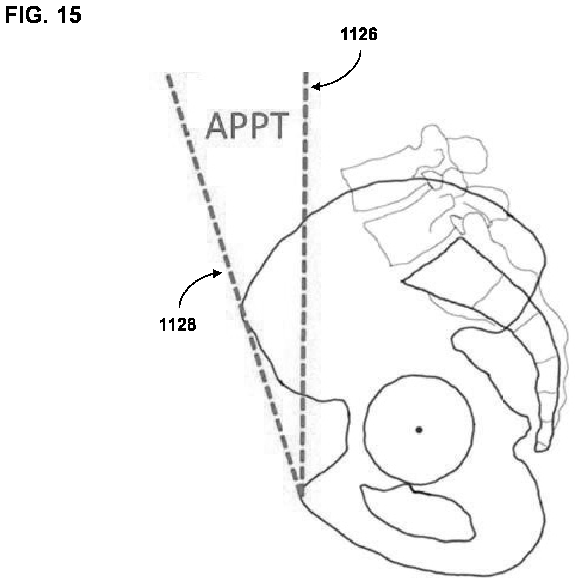

In a further embodiment, the patient's spinal alignment may be classified according to an evaluation of the Anterior Pelvic Plane Tilt (APPT) in the standing position (illustrated in **FIG. 15****).** This may be performed in addition to, or instead of the evaluation of PI-LL mismatch in process 912. A neutral APPT (i.e., APPT angle close to 0° with respect to the vertical gravity axis, e.g., 0° to 10°) reflects a normal spine alignment, and may support a classification of "normal spine alignment" ("1"), while a significant anterior or posterior APPT angle indicates a flatback deformity, supporting a classification of "flat back deformity" ("2").

The spine mobility classifications (A, B) and spine alignment classifications (1, 2) and associated pathologies and criteria are summarized in **Table 1,** below.

**Table 1: Hip-spine classification in THA**

| **Class identification** | **Pathology classification** | **Criteria** |
|---|---|---|
| 1 | Normal spinal alignment | Defined as PI-LL ± 10° |
| 2 | Flatback deformity | Defined as PI-LL > 10° |
| A | Normal spine mobility | Defined as ≥ 10° change in SS angle from stand to sit |
| B | Stiff spine mobility | Defined as < 10° change in SS angle from stand to sit |

Following processes 904 and 912, the patient is assigned a combined classification of 1A, 1B, 2A, or 2B, described in **Table 2** (below) based on their spinal mobility classification and spine alignment classification.

Process 920 uses the combined classification resulting from, e.g., parallel processes 904, 912, to plan the placement of the acetabular shell prosthesis, including defining the planned or target orientation of the shell based on the obtained patient-specific combined classification. For patients in combined classes 1A, 1B, and 2A, a direct determination of the acetabular shell orientation can be proposed to the user. For patients in combined class 2B, an additional evaluation of posterior pelvic tilt is performed in order to yield a proposed acetabular shell orientation. Process 920 includes subprocesses 922, 924, and 926.

Subprocess 922 defines a default orientation of the acetabular shell prosthesis for patients having a combined classification of 1A or 2A. For such patients, the default orientation is defined by an inclination angle of 40° and an anteversion angle of 20-25°, based on the patient's 1A or 2A combined classification.

Subprocess 924 defines a default orientation of the acetabular shell prosthesis for patients having a combined classification of 1B. For such patients, the default orientation is defined by an inclination angle of 45° and an anteversion angle of 25-30°, based on the patient's 1B classification.

For patients having a combined classification of 2B, subprocess 926 evaluates the patient's Posterior Pelvic Tilt (PPT) on the 2D image of the patient in the standing position. Once the PPT angle is measured, patients are assigned a PPT sub-classification of less than or equal to (≤) 13° (PPT ≤ 13°), or greater than (>) 13° (PPT > 13°).

For patients having a combined classification of 2B and a PPT ≤ 13°, subprocess 922 defines a default orientation of the acetabular shell prosthesis by an inclination angle of 40° and an anteversion angle of 20-25°, based on the 2B combined classification the PPT ≤ 13°.

For patients having a combined classification of 2B and a PPT > 13°, subprocess 924 defines a default orientation of the acetabular shell prosthesis by an inclination angle of 45° and an anteversion angle of 25-30°, based on the 2B combined classification and the PPT > 13°.

**Table 2: Combined classifications and corresponding optimal acetabular shell orientations**

| **Class ID** | **Pathology classification** | **Explanation of pathology** | **Proposed target shell orientation** |
|---|---|---|---|
| 1A | Normal spinal alignment; Normal spine mobility | Normal | (Native anatomy) |
| | | | Inclination 40° |
| | | | Anteversion 20-25° |
| 1B | Normal spinal alignment; Stiff spine mobility | "Stuck standing" Stiff spine needs more inclination and anteversion | Inclination 45° |
| | | | Anteversion 25-30° |
| 2A | Flatback deformity; Normal spine mobility | Anterior pelvic tilt from the hip flexion contracture will resolve post-operative alignment | (Native anatomy) |
| | | | Inclination 40° |
| | | | Anteversion 20-25° |
| 2B | Flatback deformity; Stiff spine mobility | "Stuck sitting" Spinal deformity will cause more functional cup anteversion | 1) If posterior pelvic tilt ≤ 13°, then: (Native anatomy) Inclination 40° Anteversion 20-25° 2) If posterior pelvic tilt > 13°, then: Inclination 45° Anteversion 25-30° |

Workflow 900 may provide as output 928 the combined classification of the patient, e.g., 1A, 1B, 2A, or 2B, thereby facilitating identification and classification of patients having spinal deformities and abnormal spino-pelvic mobilities. As described above, the output of workflow 900 is also based on as few as two pre-operative 2D images, and several automatic onscreen spino-pelvic parameter measurements that can be performed pre-operatively as described further below. Accordingly, the use of workflow 900 has no impact on intraoperative time.

Additionally, workflow 900 may provide as output a default acetabular shell orientation based on the individual patient's combined classification. This default orientation may be displayed to the surgeon, e.g., on a display device as described herein, e.g., display device 110 **(****FIG. 3****),** XR headset 150 **(****FIG. 4****),** or another display device in communication with computer platform 400. The default orientation may subsequently be manually adjusted by the surgeon if or as desired, either pre-operatively or intra-operatively.

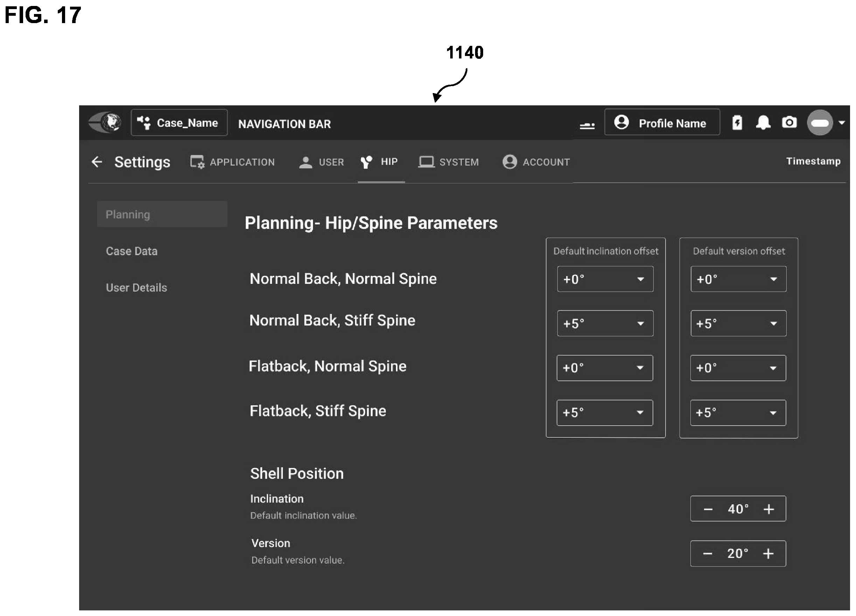

In one embodiment, the proposed acetabular shell inclination and version offsets (with respect to native anatomy) can be configured as a preset on the user preference settings page 1140 of the software application, as shown in **FIG. 17****.** Depending on the pathology detected and classified by the workflow 900, the planning software can automatically suggest the appropriate offsets to the acetabular shell placement. The user is provided with the ability to modify (if necessary) the suggested plan, and is then prompted to confirm the proposed acetabular shell planning offsets before proceeding with the intra-operative acetabular shell placement, which may be performed with the aid of surgical system 10 and optionally, surgical robot 100.

Turning next to **FIGS. 10-11****,** various subprocesses and aspects of process 902 are shown, in which the 2D images used in workflow 900 **(****FIG. 9****)** are processed on a computer platform such as, e.g., platform 400 **(****FIG. 4****)** for downstream use in surgical planning.

Process 930 identifies bone landmarks on the pre-operative 2D images, and includes subprocesses 932, 934, 936, 938, 940, and 942. Subprocess 932 includes importing the pre-operative 2D images, e.g., the x-ray images of the patient in the standing and seated positions, to the computer platform, e.g., computer platform 400 **(****FIG. 4****).** The images may be imported, e.g. in DICOM image format from an imaging device 420 **(****FIG. 4****)** such as, e.g., an image database, a C-arm imaging device, or other imaging device, using the software interface.

Subprocess 934 evaluates the quality of the 2D images including, e.g., pixel resolution, image size, contrast, brightness, and orientation. Each parameter may be automatically or manually reviewed with respect to the 2D images, and visualization parameters may be adjusted if desired by the user using control sliders on the user interface to improve image quality.

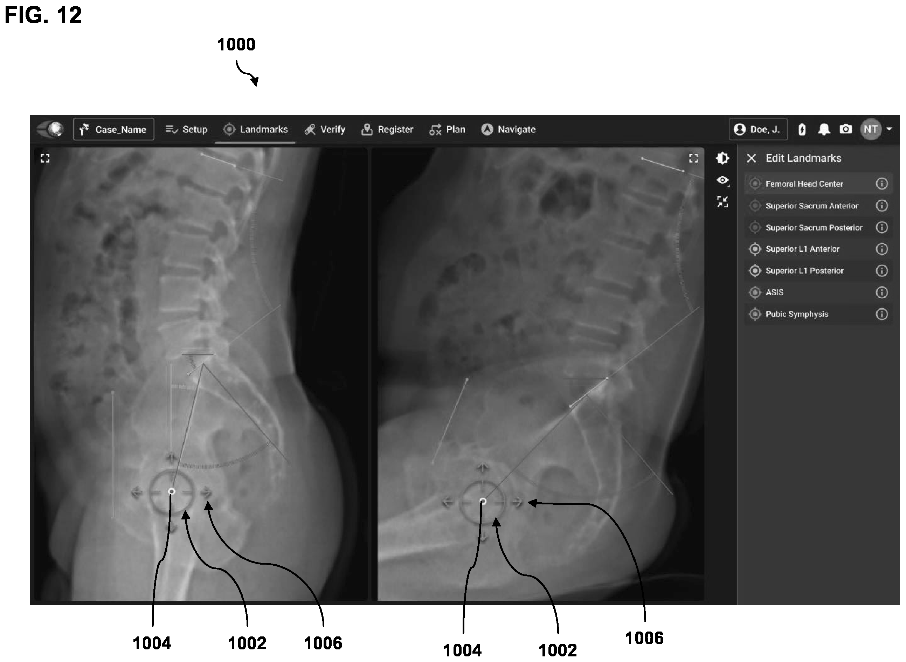

Landmarks may then be identified in each 2D image via either of subprocess 936 or 938. Subprocess 936 automatically detects the position of known anatomical (bone) landmarks, e.g., in lateral standing, lateral sitting, and anterior-posterior (AP) standing images using the computer platform, e.g., computer platform 400, in communication with a trained neural network (e.g., via machine learning algorithms). In subprocess 938, a user may alternatively identify the bone landmarks manually on the pre-operative 2D images, e.g., using a touchscreen of an intra-operative device, a laptop, or other computing device. In certain embodiments, the landmark identification user interface 1000 may provide a crosshair/reticle icon 1002 capable of being dragged and dropped onto the target region of interest to mark a single point location 1004, as illustrated in **FIG. 12****.**

Regardless of whether the bone landmarks were identified via subprocess 936 or 938, subprocess 940 provides a user interface for manually editing or correcting the location(s) of the landmark(s) identified previously via automatic or manual processes. The user interface provides the ability to visualize the locations of landmarks on the 2D images, separately edit each landmark location, and edit the location with a high resolution controller in the two directions of a plane (e.g., X, Y axes). Manual edits or corrections may be performed by a user by interacting with a touchscreen of an intra-operative device, a laptop, or other computing device as in subprocess 938. In certain embodiments, with reference to **FIG. 12****,** the user may adjust the position of each identified landmark by using or actuating an arrow 1006 around the crosshair/reticle icon 1002 associated with a particular point location 1004 to be edited. Actuation of an arrow 1006 may shift the position of the crosshair/reticle icon 1002 in the direction of the arrow, e.g., in the selected direction. Other methods of user input are may also be used, e.g., drag and drop, or use of a keyboard or other input device.

Once corrected, the locations of the landmarks on the 2D images may be stored in a data storage device 944 **(****FIGS. 10-11****).** Subprocess 942 provides a user interface for manually confirming the location(s) of the landmark(s) identified and corrected via subprocesses 936, 938, and 940.

After bone landmarks are identified according to process 930, process 950 analyzes the pre-operative 2D images with bone landmarks identified thereon to determine various spino-pelvic alignment parameters used in workflow 900 to determine the patient's spinal alignment and mobility classification.

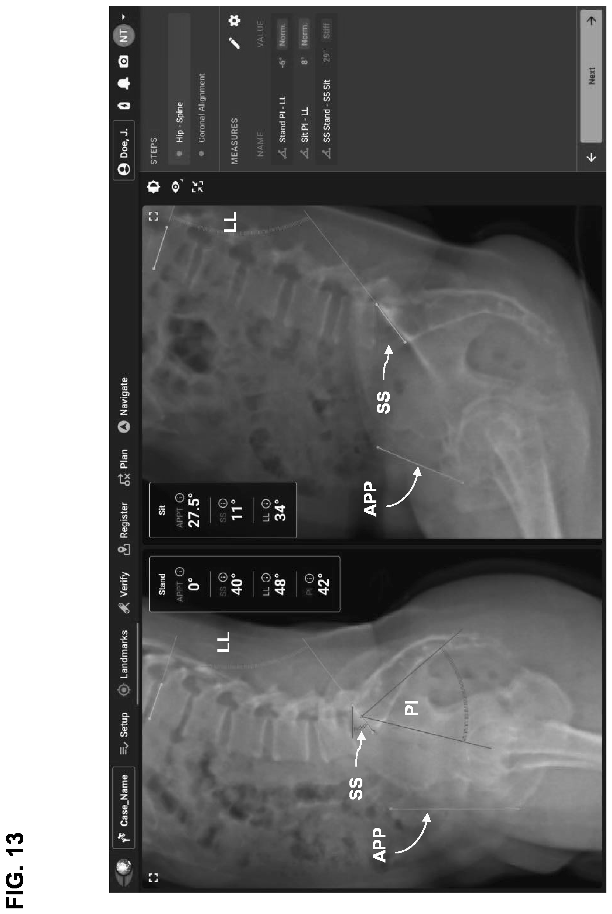

Anatomical reference lines, e.g., two or more anatomical reference lines are created or drawn on the 2D images, connecting the previously identified landmarks. Angular measurements between the anatomical reference lines are automatically computed and displayed to the user, e.g., on a display device as described elsewhere herein. In an exemplary embodiment, one or more reference lines, angles, and computed angular measurements (e.g., spino-pelvic alignment parameters) are displayed to the user in **FIG. 13****.** As shown, the reference line(s) and angles are displayed on a lateral standing (left) and lateral sitting image (right) of the patient, and the angular measurements (e.g., spino-pelvic alignment parameters LL, PI, PI-LL, SS, and APPT) are quantified. These measurements correspond to spino-pelvic alignment parameters that are subsequently used as inputs in processes of workflow 900 **(****FIG. 9****),** described previously.

Process 950 may include subprocesses 952, 954, and 956. Subprocess 952 determines a pelvis imbalance on an anterior-posterior (AP) view image of the patient in the standing position; subprocess 954 determines spino-pelvic alignment parameters on a lateral view image of the patient in the standing position; and subprocess 956 determines spino-pelvic alignment parameters on a lateral view image of the patient in the seated position. The determinations in subprocesses 952, 954, and 956, and particularly subprocesses 954 and 956, may then be used to determine the specific spino-pelvic parameters used as inputs in workflow 900 or other surgical planning workflows.

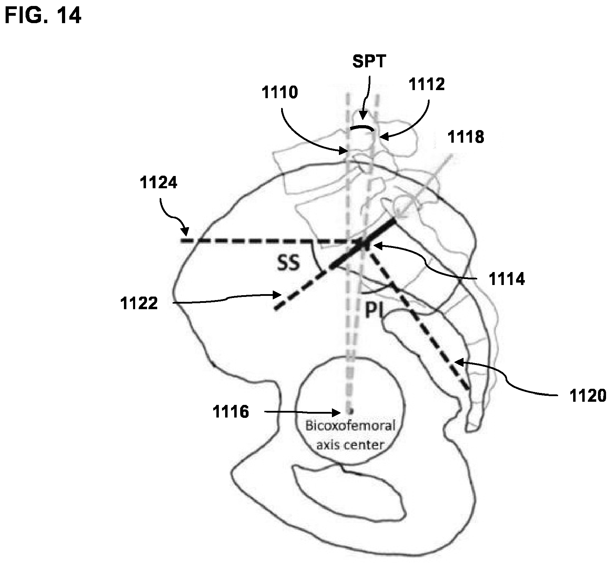

One spino-pelvic alignment parameter to be determined is the spino-pelvic tilt (SPT) angle, illustrated in **FIG. 14****.** This parameter is measured, e.g., on a lateral view image of the patient in the standing and seated positions, between the vertical line (i.e., gravity vector) 1110 and a line 1112 running from the midpoint 1114 of the sacral endplate 1118 to the center of the bicoxofemoral axis 1116, i.e., the axis joining the two femoral head centers. The two lines are projected on the sagittal plane (labeled in **FIGS. 7-8****),** as shown in **FIG. 14****.** Once projected, the SPT angle between the lines 1110 and 1112 may be automatically computed.

A further spino-pelvic alignment parameter to be determined is the pelvic incidence (PI) angle. This parameter is measured, e.g., on a lateral view image of the patient in the standing and seated positions. With continued reference to **FIG. 14****,** The PI angle is measured between a line 1120 perpendicular to the sacral endplate 1118 at its midpoint 1114, and a line 1112 connecting the same point to the center of the bicoxofemoral axis 1116. The two lines 1112, 1120 are projected on the sagittal plane. Once projected, the PI angle between the lines 1112 and 1120 may be automatically computed.

A further spino-pelvic alignment parameter to be determined is the sacral slope (SS) angle, or sacral tilt (ST). This parameter is measured, e.g., on a lateral view image of the patient in the standing and seated positions. With continued reference to **FIG. 14****,** the SS angle is measured between a line 1122 parallel to the sacral endplate 1118 and a horizontal reference line 1124 projected on the sagittal plane. Once projected, the SS angle between the lines 1122 and 1124 may be automatically computed. The automatically computed difference between the SS angle from standing to sitting position is used as an input in process 904 of workflow 900.

With reference to **FIG. 15****,** a further spino-pelvic alignment parameter to be determined is the anterior pelvic plane tilt (APPT) angle. This parameter is measured, e.g., on a lateral view image of the patient in the standing and seated positions. The APPT angle is measured between the vertical line 1126 (i.e., gravity vector) and the anterior pelvic plane (APP) 1128 projected on the sagittal plane. Once projected, the APPT angle between the gravity vector 1126 and projected APP 1128 may be automatically computed. A positive value APPT angle represents an anterior pelvic tilt, whereas a negative value APPT angle represents a posterior pelvic tilt. The automatically computed APPT angle is used as an input in subprocess 926 (process 920) of workflow 900.

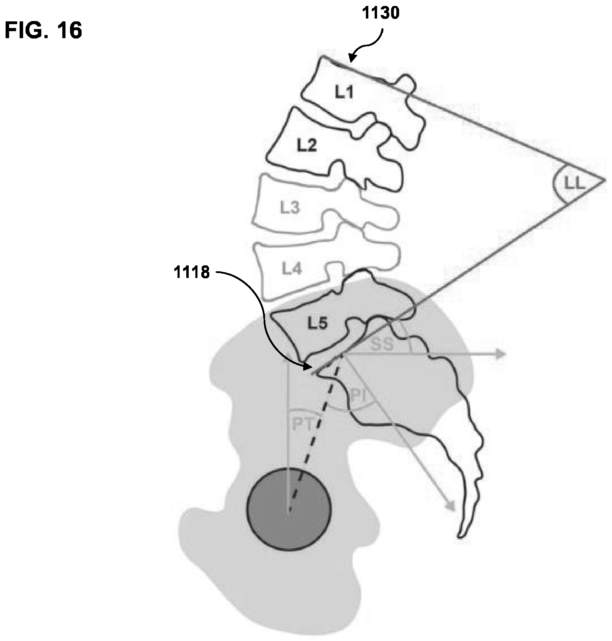

With reference to **FIG. 16****,** a further spino-pelvic alignment parameter to be determined is the pelvic incidence-lumbar lordosis angle mismatch (PI-LL). This parameter is measured, e.g., on a lateral view image of the patient in the standing and seated position. The PI-LL mismatch is measured as the difference between the Pelvic Incidence (PI) and Lumbar Lordosis (LL) angles. The LL angle is measured between the sacral endplate 1118 and the superior surface 1130 of the L1 vertebra. The PI angle is measured as described above with reference to **FIG. 14****.** The PI and LL angles, and the difference between them, may be automatically computed, and the PI-LL angle mismatch is used as an input in process 912 of workflow 900.

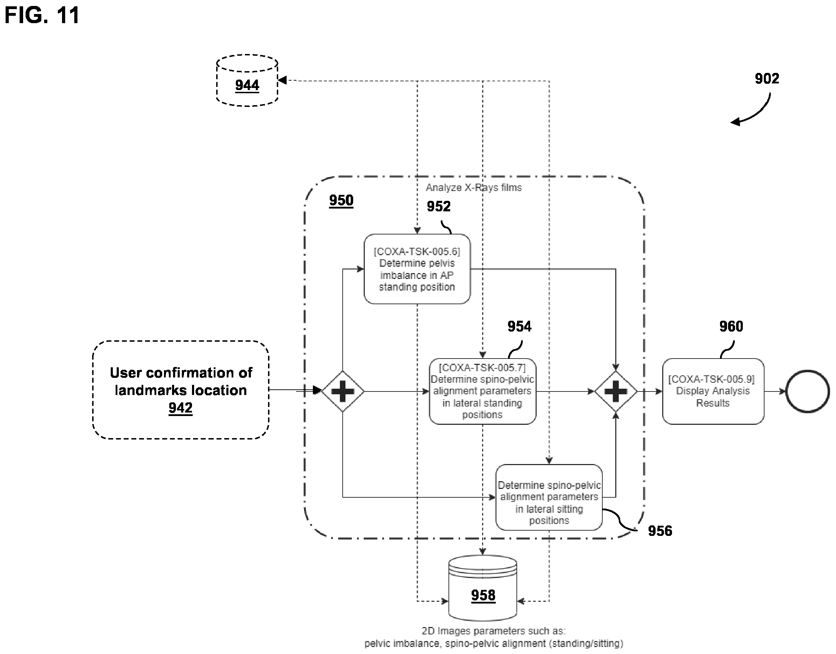

Upon computation of spino-pelvic alignment parameters as described herein, the parameters may be stored in a data storage device 958 **(****FIG. 11****).** The spino-pelvic alignment parameters are used as inputs in, e.g., processes 904, 912, 920 of workflow 900 as described herein, for planning the placement of the acetabular shell prosthesis. Additionally, process 960 may display the results of the analyzing, e.g., the computed spino-pelvic alignment parameters including angular measurements, on a display in communication with the computer platform 400, as shown in **FIG. 13****.**

### Further Definitions and Embodiments

In the above description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways.

It is to be understood that the phraseology and terminology used herein is for the purpose of description and should not be regarded as limiting. The use of "comprise," "comprising," "comprises," "include," "including," "includes," "have," "has," "having," or variations thereof, is meant to encompass the items listed thereafter and equivalents thereof as well as additional items in an openended manner. The use of such terms includes one or more stated features, integers, elements, steps, components, or functions, but does not preclude the presence or addition of one or more other features, integers, elements, steps, components, functions, or groups thereof.

Furthermore, as used herein, the common abbreviation "e.g.," which derives from the Latin phrase "exempli gratia," may be used to introduce or specify a general example or examples of a previously mentioned item, and is not intended to be limiting of such item. The common abbreviation "i.e.," which derives from the Latin phrase "id est," may be used to specify a particular item from a more general recitation.

The invention could be defined inter alia by the following examples:
1. A system for planning a navigated total hip arthroplasty (THA) procedure on a patient, comprising a computer platform including a processor and a memory, operative to: obtain a pre-operative 2D image of the patient in each of a standing position and a seated position; evaluate a change in sacral slope (SS) in the 2D images from the standing position to the seated position, to assign a spinal mobility classification of: normal spine mobility (A), or stiff spine mobility (B); evaluate a Pelvic Incidence-Lumbar Lordosis (PI-LL) angle mismatch on the 2D image of the standing position, to assign a spine alignment classification of: normal spine alignment (1), or flat back deformity alignment (2); and plan placement of an acetabular shell prosthesis based on the spinal mobility classification and the spine alignment classification.
2. The system of Example 1, wherein the pre-operative 2D images are lateral views.
3. The system of Example 1, wherein the spinal mobility classification of normal spine mobility (A) reflects a change in SS of at least 10° from the standing position to the seated position; the spinal mobility classification of stiff spine mobility (B) reflects a change in SS of less than 10° from the standing position to the seated position.
4. The system of Example 1, wherein the spinal alignment classification of normal spine alignment (1) reflects a PI-LL angle mismatch in a range of -10° to +10° (PI-LL ± 10°), and the spinal alignment classification of flat back deformity alignment (2) reflects a PI-LL angle mismatch greater than (>) 10°.
5. The system of Example 1, wherein the computer platform is further operative to assign to the patient a combined classification based on the spinal mobility classification and the spine alignment classification, wherein the combined classification is selected from: 1A, 1B, 2A, and 2B.
6. The system of Example 5, wherein the computer platform is further operative to plan the placement by: defining a default orientation of the acetabular shell prosthesis by an inclination angle of 40° and an anteversion angle of 20-25°, based on the patient having a combined classification of 1A or 2A.
7. The system of Example 5, wherein the computer platform is further operative to plan the placement by: defining a default orientation of the acetabular shell prosthesis by an inclination angle of 45° and an anteversion angle of 25-30°, based on the patient having a combined classification of 1B.
8. The system of Example 5, wherein the patient has a combined classification of 2B, and the computer platform is further operative to evaluate a Posterior Pelvic Tilt on the 2D image of the standing position, to assign a posterior pelvic tilt classification of less than or equal to (≤) 13°, or greater than (>) 13°.
9. The system of Example 8, wherein the computer platform is further operative to plan the placement by: defining a default orientation of the acetabular shell prosthesis by an inclination angle of 40° and an anteversion angle of 20-25°, based on the patient having the combined classification of 2B and the posterior pelvic tilt of less than or equal to (≤) 13°, or defining a default orientation of the acetabular shell prosthesis by an inclination angle of 45° and an anteversion angle of 25-30°, based on the patient having the combined classification of 2B and the posterior pelvic tilt of greater than (>) 13°.
10. The system of Example 1, wherein the computer platform is further operative to process the pre-operative 2D images of the patient by: identifying bone landmarks on the pre-operative 2D images; analyzing the pre-operative 2D images; and displaying results of the analyzing on a display in communication with the computer platform.
11. The system of Example 10, wherein the computer platform is further operative to process the pre-operative 2D images by: identifying the bone landmarks automatically using a trained neural network, or receiving user input to manually identify the landmarks on the pre-operative 2D images.
12. The system of Example 11, wherein the computer platform is further operative to identify the bone landmarks by: receive user input to manually confirm or correct a location of each of the bone landmarks.
13. The system of Example 10, wherein the computer platform is further operative to analyze the pre-operative 2D images by: drawing two or more anatomical reference lines based on the bone landmarks, and measuring angular measurements between the reference lines.
14. The system of Example 10, wherein the computer platform is further operative to analyze the pre-operative 2D images by: determining a spino-pelvic alignment parameter from the pre-operative 2D images.
15. The system of Example 14, wherein the spino-pelvic parameter comprises: a spino-pelvic tilt (SPT) angle, and the 2D images are lateral views of the patient in the standing position and the seated position.
16. The system of Example 14, wherein the spino-pelvic parameter comprises a pelvic incidence (PI) angle, and the 2D images are lateral views of the patient in the standing position and the seated position.
17. The system of Example 14, wherein the spino-pelvic parameter comprises a sacral slope (SS) angle, and the 2D images are lateral views of the patient in the standing position and the seated position.
18. The system of Example 14, wherein the spino-pelvic parameter comprises an anterior pelvic plane tilt (APPT) angle, and the 2D images are lateral views of the patient in the standing position and the seated position.
19. The system of Example 14, wherein the spino-pelvic parameter comprises pelvic incidence-lumbar lordosis (PI-LL) angle mismatch, and the 2D images are lateral views of the patient in the standing position and the seated position.
20. The system of Example 14, wherein the computer platform is further operative to use the spino-pelvic alignment parameter as an input for planning the placement of the acetabular shell prosthesis.

## Claims

1. A system for planning a navigated total hip arthroplasty (THA) procedure on a patient, comprising a computer platform including a processor and a memory, operative to:
- obtain a pre-operative 2D image of the patient in each of a standing position and a seated position;
- evaluate a change in sacral slope (SS) in the 2D images from the standing position to the seated position, to assign a spinal mobility classification of: normal spine mobility (A), or stiff spine mobility (B);
- evaluate a Pelvic Incidence-Lumbar Lordosis (PI-LL) angle mismatch on the 2D image of the standing position, to assign a spine alignment classification of: normal spine alignment (1), or flat back deformity alignment (2); and
- plan placement of an acetabular shell prosthesis based on the spinal mobility classification and the spine alignment classification.

2. The system of claim 1, wherein the pre-operative 2D images are lateral views.

3. The system of claim 1 or 2, wherein the spinal mobility classification of normal spine mobility (A) reflects a change in SS of at least 10° from the standing position to the seated position; and the spinal mobility classification of stiff spine mobility (B) reflects a change in SS of less than 10° from the standing position to the seated position.

4. The system of any one of the preceding claims, wherein the spinal alignment classification of normal spine alignment (1) reflects a PI-LL angle mismatch in a range of -10° to +10° (PI-LL ± 10°), and the spinal alignment classification of flat back deformity alignment (2) reflects a PI-LL angle mismatch greater than (>) 10°.

5. The system of any one of the preceding claims, wherein the computer platform is further operative to assign to the patient a combined classification based on the spinal mobility classification and the spine alignment classification, wherein the combined classification is selected from: 1A, 1B, 2A, and 2B.

6. The system of claim 5, wherein the computer platform is further operative to plan the placement by: defining a default orientation of the acetabular shell prosthesis by an inclination angle of 40° and an anteversion angle of 20-25°, based on the patient having a combined classification of 1A or 2A.

7. The system of claim 5 or 6, wherein the computer platform is further operative to plan the placement by: defining a default orientation of the acetabular shell prosthesis by an inclination angle of 45° and an anteversion angle of 25-30°, based on the patient having a combined classification of 1B.

8. The system of any one of claims 5-7, wherein the patient has a combined classification of 2B, and the computer platform is further operative to evaluate a Posterior Pelvic Tilt on the 2D image of the standing position, to assign a posterior pelvic tilt classification of less than or equal to (≤) 13°, or greater than (>) 13°.

9. The system of claim 8, wherein the computer platform is further operative to plan the placement by: defining a default orientation of the acetabular shell prosthesis by an inclination angle of 40° and an anteversion angle of 20-25°, based on the patient having the combined classification of 2B and the posterior pelvic tilt of less than or equal to (≤) 13°, or defining a default orientation of the acetabular shell prosthesis by an inclination angle of 45° and an anteversion angle of 25-30°, based on the patient having the combined classification of 2B and the posterior pelvic tilt of greater than (>) 13°.

10. The system of any one of the preceding claims, wherein the computer platform is further operative to process the pre-operative 2D images of the patient by:
- identifying bone landmarks on the pre-operative 2D images;
- analyzing the pre-operative 2D images; and
- displaying results of the analyzing on a display in communication with the computer platform.

11. The system of claim 10, wherein the computer platform is further operative to process the pre-operative 2D images by:
- identifying the bone landmarks automatically using a trained neural network, or
- receiving user input to manually identify the landmarks on the pre-operative 2D images and wherein preferably the computer platform is further operative to identify the bone landmarks by: receive user input to manually confirm or correct a location of each of the bone landmarks.

12. The system of any one of claims 10-11, wherein the computer platform is further operative to analyze the pre-operative 2D images by:
- drawing two or more anatomical reference lines based on the bone landmarks, and
- measuring angular measurements between the reference lines.

13. The system of any one of claims 10-12, wherein the computer platform is further operative to analyze the pre-operative 2D images by: determining a spino-pelvic alignment parameter from the pre-operative 2D images.

14. The system of claim 13, wherein the spino-pelvic parameter comprises: a spino-pelvic tilt (SPT) angle, and the 2D images are lateral views of the patient in the standing position and the seated position and wherein preferably the spino-pelvic parameter comprises a pelvic incidence (PI) angle, and the 2D images are lateral views of the patient in the standing position and the seated position and wherein preferably the spino-pelvic parameter comprises a sacral slope (SS) angle, and the 2D images are lateral views of the patient in the standing position and the seated position and wherein preferably the spino-pelvic parameter comprises an anterior pelvic plane tilt (APPT) angle, and the 2D images are lateral views of the patient in the standing position and the seated position and wherein preferably spino-pelvic parameter comprises pelvic incidence-lumbar lordosis (PI-LL) angle mismatch, and the 2D images are lateral views of the patient in the standing position and the seated position.

15. The system of claim 14, wherein the computer platform is further operative to use the spino-pelvic alignment parameter as an input for planning the placement of the acetabular shell prosthesis.
